(19) Europäisches Patentamt
European Patent Office
Office européen des brevets

(11) **EP 3 404 414 B1**

(12) **EUROPÄISCHE PATENTSCHRIFT**

(45) Veröffentlichungstag und Bekanntmachung des Hinweises auf die Patenterteilung:
**29.01.2020 Patentblatt 2020/05**

(21) Anmeldenummer: **17171487.6**

(22) Anmeldetag: **17.05.2017**

(51) Int Cl.:
***G01N 33/68*** (2006.01)

(54) **VERFAHREN UND SYSTEM ZUR DETEKTION VON SAUREM GLIAFASERPROTEIN (GFAP) IN REIFGEBORENEN UND FRÜHGEBORENEN**

METHOD AND SYSTEM FOR THE DETECTION OF ACIDIC GLIAL FIBER PROTEIN (GFAP) IN NEWBORNS AND PREMATURE INFANTS

PROCÉDÉ ET SYSTÈME DE DÉTECTION DE PROTÉINE ACIDE FIBRILLAIRE GLIALE (GFAP) CHEZ LES NOUVEAUX-NÉS ET LES PRÉMATURÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.11.2018 Patentblatt 2018/47**

(73) Patentinhaber: **Jensen, Arne**
**44797 Bochum (DE)**

(72) Erfinder: **Jensen, Arne**
**44797 Bochum (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:

- **LV HONGYAN ET AL: "Neonatal hypoxic ischemic encephalopathy-related biomarkers in serum and cerebrospinal fluid", CLINICA CHIMICA ACTA, Bd. 450, 23. Oktober 2015 (2015-10-23), Seiten 282-297, XP029297207, ISSN: 0009-8981, DOI: 10.1016/J.CCA.2015.08.021**

- **AMANDA STEWART ET AL: "445: Serum glial fibrillary acidic protein levels are significantly elevated in preterm neonates with neurologic morbidity and mortality", AMERICAN JOURNAL OF OBSTETRICS & GYNECOLOGY, Bd. 204, Nr. Suppl. 1, 1. Januar 2011 (2011-01-01), Seite S179, XP055399782, US ISSN: 0002-9378, DOI: 10.1016/j.ajog.2010.10.463**

- **KUCZIUS THORSTEN ET AL: "Simultaneous detection of three CNS indicator proteins in complex suspensions using a single immuno-PCR protocol", ANALYTICAL BIOCHEMISTRY, Bd. 431, Nr. 1, 1. Dezember 2012 (2012-12-01), Seiten 4-10, XP028951542, ISSN: 0003-2697, DOI: 10.1016/J.AB.2012.08.029**

- **THORSTEN KUCZIUS ET AL: "High sensitivity detection of the glial fibrillary acidic protein as indicator for TSE risk material in meat products using an immuno-PCR", MOLECULAR NUTRITION & FOOD RESEARCH, Bd. 53, Nr. 10, 1. Oktober 2009 (2009-10-01), Seiten 1329-1335, XP055398313, DE ISSN: 1613-4125, DOI: 10.1002/mnfr.200800587**

- **NIEMEYER C M ET AL: "Immuno-PCR: high sensitivity detection of proteins by nucleic acid amplification", TRENDS IN BIOTECHNOLOGY, ELSEVIER PUBLICATIONS, CAMBRIDGE, GB, Bd. 23, Nr. 4, 1. April 2005 (2005-04-01), Seiten 208-216, XP027778077, ISSN: 0167-7799 [gefunden am 2005-04-01]**

- MARKS K-A: "Hypoxic-ischemic brain injury and neuroprotection in the newborn infant", FUTURE NEUROLOGY, FUTURE MEDICINE, GB, Bd. 8, Nr. 3, 1. Mai 2013 (2013-05-01), Seiten 303-319, XP009195237, ISSN: 1479-6708, DOI: 10.2217/FNL.13.5 [gefunden am 2013-05-03]
- GROW JENNIFER ET AL: "Pathogenesis of hypoxic-ischemic cerebral injury in the term infant: current concepts", CLINICS IN PERINATOLOGY,, Bd. 29, Nr. 4, 1. Dezember 2002 (2002-12-01), Seiten 585-602, XP009195257, ISSN: 0095-5108, DOI: 10.1016/S0095-5108(02)00059-3
- CHARANJIT KAUR ET AL: "Hypoxic damage to the periventricular white matter in neonatal brain: role of vascular endothelial growth factor, nitric oxide and excitotoxicity", JOURNAL OF NEUROCHEMISTRY, Bd. 98, Nr. 4, 1. August 2006 (2006-08-01), Seiten 1200-1216, XP055398634, NEW YORK, NY, US ISSN: 0022-3042, DOI: 10.1111/j.1471-4159.2006.03964.x
- Anonymous: "Microcephaly - Wikipedia", , 4. Mai 2017 (2017-05-04), Seiten 1-11, XP055399776, Gefunden im Internet: URL:https://en.wikipedia.org/w/index.php?title=Microcephaly&oldid=778693829 [gefunden am 2017-08-21]
- BARBU D ET AL: "Evidence of fetal central nervous system injury in isolated congenital heart defects: microcephaly at birth", AMERICAN JOURNAL OF OBSTETRICS & GYNECOLOGY, MOSBY, ST LOUIS, MO, US, Bd. 201, Nr. 1, 1. Juli 2009 (2009-07-01), Seiten 43.e1-43.e7, XP026250091, ISSN: 0002-9378, DOI: 10.1016/J.AJOG.2009.03.029 [gefunden am 2009-05-15]

**Beschreibung**

GEBIET DER ERFINDUNG

Die vorliegende Erfindung betrifft ein Verfahren zur Detektion von saurem Gliafaserprotein (GFAP) im Blut eines früh- oder reifgeborenen Säugers bei dem weiterhin kontinuierlich der Partialdruck von Stickstoffmonoxid im Atemgas des Säugers bestimmt wird.

HINTERGRUND DER ERFINDUNG

Saures Gliafaserprotein (Abk. GFAP, engl. Glial fibrillary acidic protein) ist ein Protein, welches als Hauptbestandteil der Intermediärfilamente im Cytoplasma von Gliazellen (vor allem Astrozyten) im Zentralnervensystem vorkommt. Die Funktion ist bislang nicht vollständig geklärt.

Innerhalb des Zentralnervensystems (ZNS) kommt GFAP überwiegend in Astrozyten vor und kann daher mit gewisser Sicherheit als Marker für Astrozyten verwendet werden. Wegen seines Vorkommens in Astrozyten spielt GFAP als Marker eine wesentliche Rolle bei der Diagnostik von Hirnkrankheiten wie Hirntumoren. Es wird typischerweise in glialen Tumoren (z.B. Astrozytomen, Glioblastomen, Ependymomen und einer Reihe anderer glialer Tumoren) gefunden.

GFAP wurde auch als ein Marker zur Feststellung von Gehirnerschütterungen vorgeschlagen. Bei einer Gehirnerschütterung wird GFAP durch die Blut-Hirn-Schranke ins Blut abgegeben. Dadurch kommt es bis zu einer Woche nach einer Gehirnerschütterung in messbaren Konzentrationen im Blut vor (L. Papa et al. "Performance of Glial Fibrillary Acidic Protein in Detecting Traumatic Intracranial Lesions on Computed Tomography in Children and Youth With Mild Head Trauma." In: Academic emergency medicine: official journal of the Society for Academic Emergency Medicine, Band 22, Nr. 11, November 2015, S. 1274-1282).

Aus einem ähnlichen Grund wurde GFAP auch als ein Marker zur Feststellung einer traumatischen Hirnverletzung vorgeschlagen (T. Bogoslovsky et al. "Fluid Biomarkers of Traumatic Brain Injury and Intended Context of Use", Diagnostics 2016, 6, 37).

Das Gehirn ist ein komplexes Organ und reagiert empfindlich auf äußere Einflüsse. Im Gegensatz zu Herz, Leber oder Lunge übersteht es einen Sauerstoffmangel kaum länger als acht bis zehn Minuten. Darüber hinaus ist es besonders empfindlich gegenüber Entzündungsreaktionen, Trauma und genetischen Aberrationen.

Die Ursachen für Hirnschäden bei Neu- und Frühgeborenen sind vielfältig und reichen von Durchblutungsstörungen der Plazenta über während der Geburt abgeklemmte Gefäße, Infektionen, traumatischen Geburtsereignissen und genetischen Defekten bis hin zu Beatmungsfolgen bei sehr unreifen zu früh geborenen Kindern. In Deutschland bleiben bei rund 1000 Kindern jährlich allein aufgrund eines Sauerstoffmangels unmittelbar vor, während oder nach der Geburt schwerste Hirnschäden zurück. Neugeborene mit einem Geburtsgewicht unter 1500 Gramm sind besonders gefährdet. Je nach Ausmaß der Schädigung und der betroffenen Hirnregionen können sie leichte Funktionsstörungen des Gehirns (z.B. *"minimal brain dysfunction"*, "Zappelphilipp-Syndrom") oder schwerste körperliche und weitreichende geistige Behinderungen davon tragen. Dazu gehören Bewegungsstörungen bis hin zu Lähmungen der Arme und Beine, selbst ein Zusammenhang mit dem Auftreten von Schizophrenien wird heute vermutet. Zudem kann die Kontrolle emotionaler Impulse beeinträchtigt sein, was im Erwachsenenalter eine geringere geistige Leistungsfähigkeit, Beziehungsstörungen, Ängste, Depressionen oder Schwierigkeiten in der beruflichen Anpassung verursachen kann.

Die Belastungen für die Betroffenen und ihre Angehörigen sind beträchtlich und erfordern eine enge Kooperation zwischen Geburtshelfern, Kinderärzten, Psychologen sowie Sprach- und Bewegungstherapeuten. Die für die Solidargemeinschaft entstehenden Kosten pro Geburtenjahrgang werden in Deutschland auf mindestens 500 Millionen EUR geschätzt.

Ein Sauerstoffmangel während der Geburt schädigt bei sehr früh geborenen Kindern meist die weiße Hirnsubstanz, die im Inneren des Gehirns die natürlichen Hohlräume (Ventrikel) umgibt. Durch diese sog. periventrikuläre Leukomalazie werden Nervenbahnen angegriffen, die von der Hirnrinde u.a. zu den Beinen führen, was die genannten Bewegungsstörungen verursacht. Zudem können die empfindlichen Blutgefäße des unreifen Gehirns bei Blutdruckschwankungen, die mit dem Sauerstoffmangel verbunden sind, leicht zerreißen. Blutungen in das Hirnwasser oder in die weiße Hirnsubstanz sind die Folge.

Bei reifen Kindern führt ein Sauerstoffmangel dagegen eher zu Schäden in der grauen Substanz von Hirnrinde, Zwischen- und Mittelhirn . Betroffen sind davon jedoch maximal 0,04 Prozent der Kinder, während bei 10 bis 15 Prozent aller Frühgeborenen unter 1.500 Gramm Geburtsgewicht schwere Hirnschädigungen auftreten.

Durch klinische Strategien zum Schutz der kindlichen Hirnzellen, die vor allem bei frühgeborenen Kindern Hirnschäden vermeiden sollen, wurden in den vergangenen zehn Jahren zum Teil gute Erfolge erzielt. Dabei steht zunächst die Senkung der Frühgeburtenrate im Vordergrund, zugleich aber auch, Sauerstoffmangelzustände rechtzeitig zu erkennen und entsprechend einzugreifen.

Stresssituationen für Mutter und Kind, Blutungen und in die Gebärmutter aufsteigende Infektionen steigern das Frühgeburtsrisiko. Um Risikopatientinnen rechtzeitig zu erkennen, wird daher zunächst eine umfassende Anamnese erhoben.

In einer umfangreichen Ultraschall-Reihenuntersuchung des kindlichen Gehirns nach der Geburt (ca. 5.300 Kinder) zeigte sich, dass mit abnehmender Vitalität insbesondere bei frühgeborenen Kindern Hirnblutungen in allen Schweregraden (I bis IV; vgl. Berger et al., Eur. J. Obstet. Gynaecol. Reprod. Biol. 75 (1997) 191-203) zunehmen. Als Maß für den Vitalitätszustand wird dabei der sog. Apgar-Wert (0 bis 10 Punkte) genutzt, der Herzschlag, Atmung, Muskelspannung, Hautfärbung und Reaktionsfähigkeit der Kinder berücksichtigt. Er wird eine, fünf und zehn Minuten nach jeder Geburt bestimmt und liegt bei vitalen Kindern zwischen acht und zehn Punkten. Ein niedriger Apgar-Wert spiegelt hier unter Umständen einen durch den Sauerstoffmangel hervorgerufen Schockzustand des Kindes wieder. Während bei Sauerstoffmangel im Gehirn normalerweise das sympathische Nervensystem (Teil des vegetativen Nervensystems) den Blutfluss aus anderen weniger gefährdeten Bereichen des Organismus verstärkt in lebenswichtige Organe lenkt, sind Frühgeborene nur eingeschränkt zu einer solchen Umverteilung in der Lage. Zusammen mit der Empfindlichkeit ihrer Blutgefäße gegenüber Blutdruckschwankungen führt das zu einer äußerst geringen Toleranz gegenüber Sauerstoffmangel.

Diese Ergebnisse führten zu einem Konzept der *Frühintervention*: Schon bei ersten Anzeichen eines möglichen Sauerstoffmangels wird eine Risikoabwägung vorgenommen und alle Vorbereitungen getroffen, damit das Kind bei Sauerstoffmangel (abfallende Herzfrequenz) sofort und unter optimalen Bedingungen geboren wird. Wenn das gelingt, kann es in gutem Zustand vom Kinderarzt weiter betreut werden, und das unreife Gehirn bleibt vor Schäden bewahrt.

Da sich Hirnschäden nicht in jedem Fall durch Vorsorgeprogramme oder Frühintervention verhindern lassen, werden auch therapeutische Strategien erforscht, die das kindliche Gehirn schützen und sogar regenerieren sollen, wenn der Schaden bereits eingetreten ist. Das setzt voraus, dass die Mechanismen, die zu einem Hirnschaden führen, bekannt sind.

Es wurde festgestellt, dass das Absterben von Nervenzellen im Gehirn bei Sauerstoffmangel nach Unterschreiten eines Schwellenwertes beginnt und dann in zwei Wellen erfolgt: Durch die mangelnde Durchblutung bricht der Energiestoffwechsel im Gehirn zusammen und als Reaktion auf den Sauerstoffmangel wird vermehrt Glutamat freigesetzt - einer der wichtigsten aktivierenden Botenstoffe im Gehirn. Wenn dem Ionenaustausch an der Zellmembran keine Energie mehr zur Verfügung steht, kann auch die elektrische Membranspannung an den Nervenzellen nicht aufrecht erhalten werden. Als Folge dringen über verschiedene Ionenkanäle große Mengen Kalzium in die Zellen ein. Ein Teil dieser Kanäle wird durch Glutamat gesteuert. Der exzessive Anstieg der intrazellulären Kalzium-Konzentration, der sog. calcium-overload, aktiviert verschiedene Enzyme, die schließlich die Zellen schädigen.

Wenn der akute Sauerstoffmangel vorüber ist, normalisiert sich der Energiestoffwechsel in einigen Hirnbereichen zunächst wieder. Doch wenige Stunden später setzt eine zweite Welle des Absterbens von Nervenzellen ein: Die Nervenzellen schwellen an, es werden epileptische Aktivitätsmuster in den Hirnströmen registriert, die Anzeichen einer Schädigung der Nervenzellen sind. Als Gründe dafür vermutet man u.a. Entzündungsreaktionen und ein Ungleichgewicht zwischen hemmenden und aktivierenden Botenstoffen im Gehirn, wodurch möglicherweise der programmierte Zelltod (Apoptose) in Gang gesetzt wird. Da ein beträchtlicher Teil der Zellschäden aber erst Stunden oder Tage nach dem Sauerstoffmangel zustande kommt, wird versucht, während dieses "therapeutischen Fensters" einzugreifen.

Es werden bereits unterschiedliche medikamentöse Strategien untersucht, die Hirnschäden durch Sauerstoffmangel vermindern können, wie zum Beispiel neuroprotektive, d.h. die Nervenzellen schützende Substanzen. Verwendet werden beispielsweise Flunarizin, das bei Sauerstoffmangel den unkontrollierten Einstrom von Kalzium in die Nervenzelle mindert (Kalzium-Kanal-Antagonist), und Lubeluzole, ein Gegenspieler des Glutamats (Glutamat-Antagonist). Beide Substanzen hatten bereits bei Schlaganfall positive Resultate gezeigt. Während Lubeluzole jedoch bei Neugeborenen nicht die erhoffte Wirkung hatte, scheint Flunarizin geeignet, das kindliche Gehirn vor schweren Schäden durch Sauerstoffmangel zu bewahren.

Da die Einführung jeglicher Pharmaka in der Geburtshilfe umfangreiche Unbedenklichkeitsprüfungen erfordert, wurde zugleich nach anderen, körpereigenen Substanzen mit einer ähnlich guten Wirkung gesucht. Mit der energiereichen Verbindung Kreatin, einem Stoffwechselprodukt des Organismus, zeichnen sich schon jetzt hoffnungsvolle Ergebnisse ab (R. Berger et al., Creatine protects the immature brain from hypoxic-ischemic injury, Middelanis et al., 2003, J. Soc. Gynecol. Investig. Vol. 10, No: 2, Abstract No. 290).

Ein weitere Ansatz ist die Behandlung von Früh- und Reifgeborenen mit Hirnschaden mittels Nabelschnurblut zur funktionellen Neuroregeneration (A. Jensen, "Stammzellen aus Nabelschnurblut heilen kindlichen Hirnschaden" Top Magazin Ruhr, Wissenschaft - Medizin 23(4), 80-81 (2009); A. Jensen , "Erste Therapie eines kindlichen hypoxischen Hirnschadens mit Zerebralparese nach Herzstillstand? - Heilversuch durch autologe Nabelschnurstammzell-Transplantation." Regenerative Medizin 4(1), 30-31 (2011)). In den Publikationen A. Jensen et al. "Perinatal brain damage - from neuroprotection to neuro regeneration using cord blood stem cells" Med. Klein. 98 (2003) Suppl. 2, Seiten 22-26 und C. Meier et al. "Spastic paresis after perinatal brain damage in rats is reduced by human cord blood mononuclear cells", Pediatr. Res. 2006; 59(2), Seiten 244-249 konnte gezeigt werden, dass die systemische Transplantation menschlicher mononukleärer Zellen aus Nabelschnurblut in neugeborene Ratten, die einen experimentellen Hirnschaden erlitten hatten, sowohl zur massenhaften Einwanderung dieser Zellen in die geschädigte Hirnregion als auch zur Verhinderung spastischer Paresen führte. Die Spastik, als Leitsymptom der kindlichen Zerebralparese, war in den transplantierten Ratten praktisch nicht mehr nachweisbar.

Optimal wäre jedoch eine Behandlung von Früh- und Reifgeborenen mit Hirnschaden durch *primär frisches*, nicht kryokonserviertes, *autologes* Nabelschnurblut. Dazu ist jedoch eine rasche Feststellung der Notwendigkeit der Behandlung noch im Geburtsraum erforderlich. Die Beobachtung von Sekundärindizien wie abfallende Herzfrequenz ist nicht ausreichend aussagekräftig. Es wurde aber gefunden, dass auch bei durch Sauerstoffmangel ausgelösten Hirnschäden GFAP durch die Blut-Hirn-Schranke ins Blut abgegeben wird. GFAP kann somit prinzipiell als Marker für Hirnschäden bei Neu- und Frühgeborenen verwendet werden. Allerdings sind GFAP-Bestimmungen im Blut, wie das von L. Papa (siehe oben) beschriebene Verfahren, im Blut von Früh- und Reifgeborenen nicht möglich, da zu viel Blut benötigt würde.

Aufgabe der vorliegenden Erfindung ist es somit, ein Verfahren zur Detektion von saurem Gliafaserprotein (GFAP) zur Verfügung zu stellen, das auch zur Detektion im Blut von Früh- und Reifgeborenen unmittelbar nach Geburt anwendbar und so schnell und zuverlässig durchführbar ist, dass eine Nabelschnurbluttherapie-Entscheidung getroffen werden kann im Früh- und Neugeborenen, bevor schwerere Hirnschädigungen auftreten.

Dokumente des Standes der Technik sind:

LV ET AL: "Neonatal hypoxic ischemic encephalopathy-related biomarkers in serum and cerebrospinal fluid", CLINICA CHIMICA ACTA, Bd. 450, 2015, Seiten 282-297

STEWART ET AL: "445: Serum glial fibrillary acidic protein levels are significantly elevated in preterm neonates with neurologic morbidity and mortality", AMERICAN JOURNAL OF OBSTETRICS & GYNECOLOGY, Bd. 204, Nr. Suppl. 1, Januar 2011, Seite S179

KUCZIUS ET AL: "Simultaneous detection of three CNS indicator proteins in complex suspensions using a single immuno-PCR protocol", ANAL BIOCHEM, Bd. 431, Nr. 1,2012, Seiten 4-10

KUCZIUS ET AL: "High sensitivity detection of the glial fibrillary acidic protein as indicator for TSE risk material in meat products using an immuno-PCR", MOLECULAR NUTRITION & FOOD . RESEARCH, Bd. 53, Nr. 10, 2009, Seiten 1329-1335

NIEMEYER ET AL: "Immuno-PCR: high sensitivity detection of proteins by nucleic acid amplification", TRENDS IN BIOTECHNOLOGY, Bd. 23, Nr. 4, 2005, Seiten 208-216

MARKS: "Hypoxic-ischemic brain injury and neuroprotection in the newborn infant", FUTURE NEUROL, Bd. 8, Nr. 3, 2013, 303-319

GROW ET AL: "Pathogenesis of hypoxic-ischemic cerebral injury in the term infant: current concepts", CLINICS IN PERINATOLOGY, Bd. 29, Nr. 4, 2002, Seiten 585-602

KAUR ET AL: "Hypoxie damage to the periventricular white matter in neonatal brain: role of vascular endothelial growth factor, nitric oxide and excitotoxicity", J NEUROCHEM, Bd. 98, Nr. 4, 2006,1200-1216

ZUSAMMENFASSUNG DER ERFINDUNG

Gelöst wird diese Aufgabe durch ein Verfahren zur Detektion von saurem Gliafaserprotein (GFAP) im Blut eines früh- oder reifgeborenen Säugers, bei dem das Vorhandensein von GFAP mittels PCR-verstärktem Immunassay (I-PCR) detektiert wird und weiterhin kontinuierlich der Partialdruck von Stickstoffmonoxid im Atemgas des Säugers bestimmt wird.

I-PCR wird in P.K. Metha et al. "Detection of potential microbial antigens by immuno-PCR (PCR-amplified immunoassay)", Journal of Medical Microbiology (2014), 63, 627-641 beschrieben, auf die hier vollständig Bezug genommen wird. Bei dem Verfahren wird ein zu detektierendes Antigen, gemäß der vorliegenden Erfindung GFAP, mit einem Antigen-spezifischen Antikörper, gegebenenfalls anschließend mit einem Speziesspezifischen, biotinilierten Detektionsantikörper, über Streptavidin und an eine biotinilierte DNS zu einem Antikörper-DNS-Konjugat gekoppelt. Anschließend wird die DNS mittels PCR amplifiziert.

Im Prinzip wird zwischen vier verschiedenen I-PCR-Verfahren unterschieden: direkte I-PCR, indirekte I-PCR, Sandwich I-PCR und indirekte Sandwich I-PCR. Erfindungsgemäß sind Sandwich-I-PCR- und indirekte Sandwich-I-PCR-Verfahren bevorzugt.

Gemäß einer ersten Ausführungsform der Erfindung wird GFAP im Blut eines Säugers mittels direkter I-PCR detektiert, wobei das Blut des Säugers auf eine Mikrotiterplatte aufgebracht und anschließend biotinilierter GFAP-Detektionsantikörper zugegeben wird, der nach Entfernung ungebundenen Antikörpers weiterhin über Streptavidin an eine biotinilierte Reporter-DNS gebunden wird. Anschließend wird die DNS mittels PCR amplifiziert.

Gemäß einer zweiten Ausführungsform der Erfindung wird GFAP im Blut eines Säugers mittels indirekter I-PCR detektiert, wobei das Blut des Säugers auf eine Mikrotiterplatte aufgebracht und anschließend GFAP-Detektionsantikörper zugegeben wird, der nach Entfernung ungebundenen Antikörpers weiterhin über biotinilierten Antidetektionsantikörper, nach Entfernung ungebundenen Antikörpers, über Streptavidin an eine biotinilierte Reporter-DNS gebunden wird. Anschließend wird die DNS mittels PCR amplifiziert.

Gemäß einer dritten Ausführungsform der Erfindung wird GFAP im Blut eines Säugers mittels Sandwich-I-PCR detektiert, wobei GFAP-Captureantikörper auf einer Mikrotiterplatte immobilisiert wird, Blut des Säugers dazugegeben und anschließend biotinilierter GFAP-Detektionsantikörper zugegeben wird, der nach Entfernung ungebundenen Antikörpers weiterhin über Streptavidin an eine biotinilierte Reporter-DNS gebunden wird. Anschließend wird die DNS mittels PCR amplifiziert.

Gemäß einer vierten Ausführungsform der Erfindung wird GFAP im Blut eines Säugers mittels indirekter Sandwich-I-PCR detektiert, wobei GFAP-Captureantikörper auf einer Mikrotiterplatte immobilisiert wird, Blut des Säugers dazugegeben und anschließend GFAP-Detektionsantikörper zugegeben wird, der nach Entfernung ungebundenen Antikörpers weiterhin über biotini-lierten Antidetektionsantikörper und, nach Entfernung ungebundenen Antikörpers, über Streptavidin an eine biotinilierte Reporter-DNS gebunden wird. Anschließend wird die DNS mittels PCR amplifiziert.

Bevorzugt wird GFAP im Blut eines Säugers mittels Sandwich-I-PCR oder indirekter Sandwich-I-PCR detektiert, da die direkte Beschichtung von biologischen Proben als Antigenquelle nicht notwendig ist.

GFAP-Antikörper sind kommerziell erhältlich, beispielsweise von der Firma Dianova GmbH, Hamburg, Deutschland. Der GFAP-Antikörper kann in einer angemessenen Verdünnung in Wasser zwischen 1:100 bis 1:1000, bevorzugt zwischen 1:300 bis 1:700, wie 1:500 zugegeben werden.

Bevorzugt werden ungebundene Antikörper, ungebundenes Strepavidin und ungebundene DNS aspiriert und jeweils anschließend die Miktotiterplatten mit einer Pufferlösung, wie TBST-Puffer gewaschen.

Im Falle der indirekten I-PCR oder der indirekten Sandwich-I-PCR kann beispielsweise biotiniliertes Ziege-IgG anti-Kaninchen (goat IgG anti-rabbit) verwendet werden, das ebenfalls kommerziell erhältlich ist, beispielsweise von der Firma Dianova GmbH, Hamburg, Deutschland.

Rekombinantes Streptavidin ist ebenfalls kommerziell erhältlich, beispielsweise von der Firma Roche, Mannheim, Deutschland.

Gemäß einer ersten bevorzugten Ausführungsform der Erfindung wird GFAP im Blut eines Säugers mittels direkter I-PCR detektiert, wobei das Blut des Säugers auf eine Mikrotiterplatte aufgebracht und anschließend biotinilierter GFAP-Detektionsantikörper zugegeben wird, der nach Entfernung ungebundenen Antikörpers weiterhin an Streptavidin und, nach Entfernung überschüssigem Streptavidins, an eine biotinilierte Reporter-DNS gebunden wird. Anschließend wird die DNS, nach Entfernung überschüssiger DNS, mittels PCR amplifiziert.

Gemäß einer zweiten bevorzugten Ausführungsform der Erfindung wird GFAP im Blut eines Säugers mittels indirekter I-PCR detektiert, wobei das Blut des Säugers auf eine Mikrotiterplatte aufgebracht und anschließend GFAP-Detektionsantikörper zugegeben wird, der nach Entfernung ungebundenen Antikörpers weiterhin über biotinilierten Antidetektionsantikörper und, nach Entfernung ungebundenen Antikörpers, an Streptavidin und, nach Entfernung überschüssigem Streptavidins, an eine biotinilierte Reporter-DNS gebunden wird. Anschließend wird die DNS, nach Entfernung überschüssiger DNS, mittels PCR amplifiziert.

Gemäß einer dritten bevorzugten Ausführungsform der Erfindung wird GFAP im Blut eines Säugers mittels Sandwich-I-PCR detektiert, wobei GFAP-Captureantikörper auf einer Mikrotiterplatte immobilisiert wird, Blut des Säugers dazugegeben und anschließend biotinilierter GFAP-Detektionsantikörper zugegeben wird, der nach Entfernung ungebundenen Antikörpers weiterhin an Streptavidin und, nach Entfernung überschüssigem Streptavidins, an eine biotinilierte Reporter-DNS gebunden wird. Anschließend wird die DNS, nach Entfernung überschüssiger DNS, mittels PCR amplifiziert.

Gemäß einer vierten bevorzugten Ausführungsform der Erfindung wird GFAP im Blut eines Säugers mittels indirekter Sandwich-I-PCR detektiert, wobei GFAP-Captureantikörper auf einer Mikrotiterplatte immobilisiert wird, Blut des Säugers dazugegeben und anschließend GFAP-Detektionsantikörper zugegeben wird, der nach Entfernung ungebundenen Antikörpers weiterhin über biotinilierten Antidetektionsantikörper und, nach Entfernung ungebundenen Antikörpers, an Streptavidin und, nach Entfernung überschüssigen Streptavidins, an eine biotinilierte Reporter-DNS gebunden wird. Anschließend wird die DNS, nach Entfernung überschüssiger DNS, mittels PCR amplifiziert.

Die beschriebenen Immunoassay-Schritte können automatisiert durchgeführt werden.

Als PCR-Verfahren wird erfindungsgemäß vorzugsweise die Echtzeit-PCR (Realtime-PCR) eingesetzt. Bei der Real-Time PCR kann über Fluoreszenzfarbstoffe die Zunahme von PCR-Produkten in Echtzeit verfolgt werden. Geeignete Geräte zur Durchführung der Echtzeit-PCR sind ebenfalls kommerziell erhältlich beispielsweise unter den Handelsnamen LightCycler (der Firma Roche), LightCycler 480II (der Firma Roche), Taqman 7900HT (der Firma Life Technologies) und ViiA7 (der Firma Life Technologies).

Das erfindungsgemäße Verfahren zur Detektion von saurem Gliafaserprotein (GFAP) im Blut eines Säugers mittels I-PCR ermöglicht die zeitnahe Detektion von GFAP auch mit geringen Blutmengen und ist daher für die Detektion von GFAP im Blut von Neu- und Frühgeborenen geeignet.

Das erfindungsgemäße Verfahren zur Detektion von saurem Gliafaserprotein (GFAP) im Blut eines Säugers mittels I-PCR wird bevorzugt mit weiteren Verfahren zu einem System kombiniert, das eine erhöhte Genauigkeit bei der Erkennung von durch Sauerstoffmangel ausgelösten Hirnschäden, insbesondere bei Neu- und Frühgeborenen unmittelbar nach Geburt, erlaubt.

Gemäß der vorliegenden Erfindung wird zusätzlich zum Verfahren zur Detektion von saurem Gliafaserprotein (GFAP) im Blut eines Säugers mittels PCR-verstärktem Immunassay ein Verfahren zur Erkennung von durch Sauerstoffmangel ausgelösten Hirnschäden aus dem Atemgas in Kombination bereitgestellt. Bei der Atemgasanalyse wird die Ausatemluft eines Probanden untersucht. Es ist bekannt, dass in der ausgeatmeten Luft spezifische Krankheitsmarker und Metabolite von Medikamenten und Stoffwechselprozessen gefunden werden können.

Es wurde nun überraschend gefunden, dass bei durch Sauerstoffmangel ausgelösten Hirnschäden Stickstoffmonoxid, NO, gebildet wird, das im Atemgas des Patienten nachweisbar ist. Allerdings werden Stickoxide auch durch Entzündungen im Körper des Patienten gebildet, wie in der DE 10 130 296 B4 beschrieben. Die durch Entzündungen hervorgerufene NO-Konzentration im Atemgas ist in erster Näherung konstant, während das Anflutungsprofil von NO bei durch Sauerstoffmangel ausgelösten Hirnschäden abrupt ansteigt.

Somit ist bei der Detektion von NO zur Bestimmung von durch Sauerstoffmangel ausgelösten Hirnschäden im Atemgas eines Patienten die NO-Konzentration in Relation zu einer Basislinie zu beobachten. Zur Lösung der Aufgabe wird daher ein Verfahren zur Bestimmung von Stickstoffmonoxid im Atemgas eines Säugers zur Verfügung gestellt, bei dem ausgeatmetes Gas des Säugers kontinuierlich aufgefangen und der Partialdruck von Stickstoffmonoxid (NO) kontinuierlich gemessen wird.

Das Verfahren zur Bestimmung von Stickstoffmonoxid im Atemgas eines Säugers wird bevorzugt nach dem in der DE 10 130 296 B4 beschriebenen Verfahren durchgeführt, mit dem Unterschied, dass die Stickstoffmonoxid-Bestimmung kontinuierlich durchgeführt wird.

Für das erfindungsgemäße Verfahren zur Bestimmung von Stickstoffmonoxid im Atemgas eines Säugers wird bevorzugt eine Vorrichtung zur Messung des Partialdrucks von Stickstoffmonoxid (NO) im Atemgas eines Säugers verwendet, bestehend aus einem Gehäuse mit Gaszuführung, einem porösen, gasdurchlässigen Körper, der als Anreicherungselement dient und einem gassensitiven Sensor, der ein dem Partialdruck von NO proportionales Signal liefert, bei der der poröse gasdurchlässige Körper aus einem Trägermaterial und einem Absorptionsmittel besteht, wobei sich das Absorptionsmittel auf der Oberfläche des Trägermaterials befindet und ein selektives, reversibles Absorptionsvermögen für NO aufweist. Als Absorptionsmittel wird vorzugsweise ein Calixaren ausgewählt.

Bevorzugt werden die Verfahren kombiniert angewendet, indem vorweg ein Verfahren zur Bestimmung von Stickstoffmonoxid im Atemgas eines Säugers durchgeführt wird und anschließend das I-PCR-Verfahren angewendet wird.

Zur weiteren Risikoabschätzung und Entscheidung über die Anwendung einer Nabelschnurbluttherapie eignet sich auch die Bestimmung des Kopfumfangs der Säuger. Überraschend wurde gefunden, dass eine U-förmige Beziehung zwischen den Abweichungen der Kopfumfänge gegenüber dem Bereich zwischen der 25. und der 75. Perzentile und der Rate an WMD (White Matter Damage, %) besteht, während sich z.B. bei den Gewichts- und Längenperzentilen nur ein Anstieg der WMD-Rate unterhalb der 10. Perzentile ergibt.

Gemäß einer weiteren erfindungsgemäßen Ausführungsform der vorliegenden Erfindung wird zusätzlich zum obigen Verfahren ein Verfahren zur Bestimmung des Kopfumfangs eines Säugers in Kombination bereitgestellt.

Der Kopfumfang kann schon im Mutterleib mittels Sonographie ermittelt werden. Alternativ oder bevorzugt zusätzlich wird der Kopfumfang (Stirn-Hinterhaupt, fronto-occipital) nach der Geburt mittels eines einfachen Maßbands ermittelt.

Gemäß einer weiteren bevorzugten Ausführungsform der vorliegenden Erfindung wird zusätzlich zum Verfahren zur Detektion von saurem Gliafaserprotein (GFAP) im Blut eines Säugers mittels PCR-verstärktem Immunassay ein Verfahren zur Bestimmung des Kopfumfangs eines Säugers und ein Verfahren zur Erkennung von durch Sauerstoffmangel ausgelösten Hirnschäden aus dem Atemgas in Kombination bereitgestellt.

Die Erfindungsgemäße Aufgabe wird auch durch ein System zur Bestimmung von Hirnschäden bei Früh- und Reifgeborenen gelöst, das ein Gerät zur Detektion von saurem Gliafaserprotein (GFAP) im Blut eines Säugers mittels PCR-verstärktem Immunassay (I-PCR) und ein Gerät zur Bestimmung des Partialdrucks von Stickstoffmonoxid im Atemgas eines Säugers umfasst. Des Weiteren umfasst das erfindungsgemäße System vorzugsweise auch ein Gerät zur Bestimmung des Kopfumfangs eines Säugers.

Bevorzugt wird der Kopfumfang des Säugers mittels Schädelsonographie noch im Mutterleib und anschießend nach der Geburt mittels eines Bandmaßes bestimmt.

Bevorzugt ist die Nutzung einer Datenbank vorgesehen, in der die Daten gesammelt und die individuellen Gefährdung des Kindes zur Stellung der Transplantations-(TX)-Indikation für Stammzellen aus Nabelschnurblut evaluiert wird.

Das erfindungsgemäße System zur Erhebung von Daten erlaubt eine bislang einmalige 'realtime' Gefährdungsanalyse (Risiken in der Schwangerschaft sowie vor, während und nach der Geburt), gepaart mit zielgerichtetem und zeitgerechtem Einsatz der Nabelschnurblut-Transplantation zur Verhinderung und/oder Behandlung frühkindlicher Hirnschäden (Zerebralparesen).

Das erfindungsgemäße System zur Erhebung von Daten über Risikofaktoren und Befunde, inkl. Bildgebung, würde auch erlauben, nicht nur den Therapieerfolg zu dokumentieren, sondern auch den wirkungsvollsten Transplantationszeitpunkt (der auch experimentell noch nicht bekannt ist) zu definieren und zukünftig für die Patienten individuell zu optimieren, z.B. als 'lernendes' Experten-System durch kontinuierliche Evaluation der Nachuntersuchungsdaten.

Die Voraussetzung für einen therapeutischen Einsatz von Nabelschnurblut-Stammzellen zur Prävention und Therapie von frühkindlichen Hirnschäden, die sich zu einer Infantilen Zerebralparese entwickeln können, ist die Einlagerung bzw. Zwischenlagerung des Nabelschnurblutes zum Zeitpunkt der Geburt. Eine weitere Möglichkeit gibt es nicht.

Ziel des erfindungsgemäßen Systems ist es, mit Hilfe der gewonnenen Daten eine Patientengruppe von werdenden Müttern zu definieren, deren Kinder von einer Nabelschnurblut-Einlagerung profitieren können. Ein weiteres Ziel des erfindungsgemäßen Systems ist es, eine Gruppe von neugeborenen Kindern zu identifizieren, die von einer (kurzfristigen) Transplantation von frischem oder kryokonserviertem Nabelschnurblut profitieren.

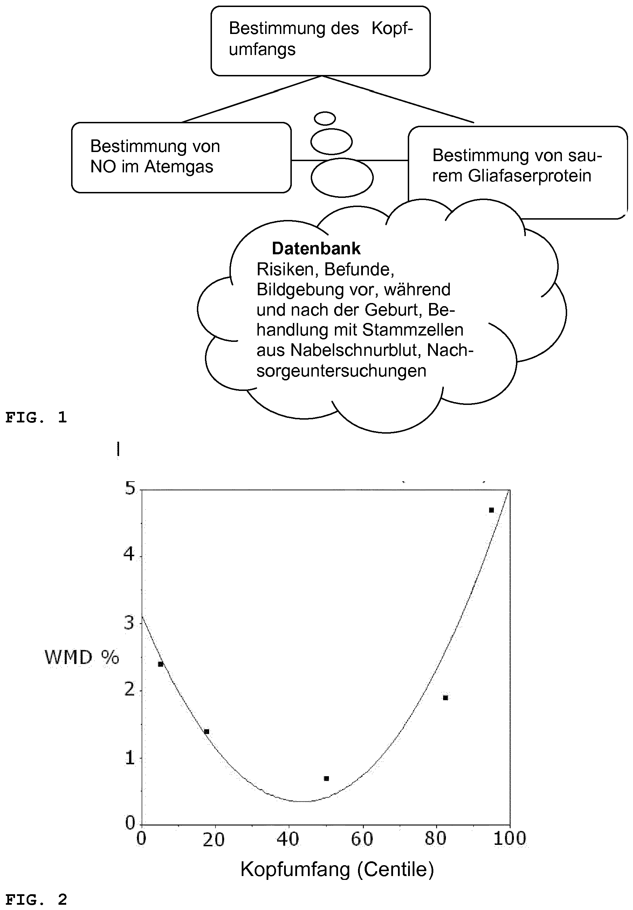

**Figur 1** zeigt das erfindungsgemäße System zur Erfassung von Daten aus dem Kopfumfang, die bevorzugt schon während der Schwangerschaft in regelmäßigen Abständen mittels Sonographie ermittelt und in einer Datenbank gespeichert werden. Der kopfumfang wird vorzugsweise auch nach der Geburt mit Hilfe eines Maßbands bestimmt. Des Weiteren werden mit dem erfindungsgemäßen System Daten zur Erfassung von saurem Gliafaserprotein im Blut des Säugers erfasst und bevorzugt einer Datenbank zugeführt, sowie die Daten einer Atemgasanalyse von Stickstoffmonoxid (NO).

**Figur 2** zeigt die Korrelation des Risikos, eine Hirnschädigung in der Weißen Substanz (White matter damage, WMD %) zu erleiden, in Abhängigkeit von den Perzentilen des Kopfumfangs zum Zeitpunkt der Geburt. Die Hirnschädigung in der Weißen Substanz (WMD) ist die neuroanatomische Grundlage für die Entstehung einer Zerebralparese. Die dargestellten Daten betreffen 4725 reife neugeborene Kinder einer prospektiven hirnsonographischen Reihenuntersuchung (geboren nach 37-43 Schwangerschaftswochen, SSW), die normalerweise nicht hirnsonographisch untersucht werden, als gemessene Datenpunkte und als Regressionskurve. Die Regressionskurve hat die Funktion:

$$y = 3,1168 - 0,12797*x + 0.0014741*x^2.$$

Die Risikoabschätzung bezüglich des Entstehens einer Hirnschädigung und damit der Notwendigkeit der Nabelschnurbluteinlagerung zum Zeitpunkt der Geburt und ggf. der Transplantation von Stammzellen aus Nabelschnurblut nach der Geburt kann auch mit Hilfe weiterer Daten erfolgen, die in der Datenbank erfasst werden. Dies sind u.a. Wachstumsretardierung des Kindes, vorzeitige Wehen, Cervixverkürzung, Makrosomie, v.a. Plazentainsuffizienz, Gemini/Mehrlinge, Alter der Mutter, Blutungen in der Schwangerschaft, Plazentationsstörungen, Gestose/Präeklampsie/HELLP-Syndrom, Gestationsdiabetes, Rh-Konstellation, CTG-Veränderungen, Nikotinabusus, Alkohol/Drogenabusus, Mütterliche Erkrankung, Infektion/Fieber/Cervicitis, Feto-fetales- Transfusions-Syndrom (FFT) bei Mehrlingen, Adipositas, Hydramnion, Beckenendlage/Querlage, Anämie, Drohende Asphyxie, Fieber unter der Geburt, Nabelschnurkomplikationen, Blutungen, Placenta praevia, Vorzeitige Plazentaablösung, Schulterdystokie, Beckenendlagenentwicklung vaginal, Vorzeitiger Blasensprung, Vaginaloperative Entbindung (Vakuumextraktion, Zangenentbindung, Forceps), Notsectio, Gerinnungsstörung, Azidose im Nabelschnurblut (pH), Erniedrigte Apgar-Werte nach 1, 5, 10 Minuten, Kindliche Reanimation, Intubation, Traumatische Geburt, Immunthrombozytopenie, Fehlbildungen, Zwerchfellhernie, Ösophagusatresie, Hydrops, Hyperbilirubinämie, Nachgewiesene Hirnblutung, Nachgewiesene Periventriculäre Leukomalazie, Neugeborenen Enzephalopathie, Hydrozephalus, Infektion, Organversagen, Multiorganversagen, Hypotonie, Spastik, Hyperexzitabilität, Cri du Chat, Ateminsuffizient, Hypoxämie, Kreislaufzentralisation.

Bevorzugt umfasst das erfindungsgemäße System eine Datenbank zur Erfassung und Verarbeitung der Daten, worin weiter bevorzugt auch noch weitere vor, während und/oder nach der Geburt gewonnene Daten erfasst werden. Die in der Datenbank erfassten Daten erlauben auch eine Prognose zur weiteren psycho-motorischen Entwicklung bei Früh- und Reifgeborenen. Dies dergestalt, dass die gewonnenen Daten zur Vorhersage des Intelligenzgrades (gemessen durch z.B. den Kramer Intelligenztest, IQ, Intelligenzquotient), der feinmotorischen und koordinativen Fähigkeiten (gemessen durch z.B. den Labyrinth-Test nach Graf & Hinton) und der neurologischen Entwicklung (gemessen durch z.B. die neurologische Untersuchung nach Touwen) ermöglichen, wie in den folgenden Beispielen (1), (2) und (3), in denen die logistische Regressionsanalyse zur Formeldarstellung verwandt wurde, beschrieben:

(1)    **IQ** = 127,782 - 1,77 * Schwangerschaftswoche + 0,012*Geburtsgewicht + 2,678 * Apgar 10' - 6,926 * White Matter Damage_vorhanden
(R= 0,462; F= 8,667; p= 0,000003)

(2)    **Labyrinthtest** = - 27,284 - 5,654 * Hirnblutung_vorhanden - 29,196 * grünes Fruchtwasser_vorhanden + 0,869 * Schwangerschaftswoche - 2,703 * White Matter Damage_vorhanden
(R= 0,508; F=10,986; p= 0,000003)

(3)    **Neurologische Untersuchung** = 86,041 - 9,137 * grünes Fruchtwasser_vorhanden - 2,534 * Geburtslage + 0,003 * Geburtsgewicht - 1,902 * Hirnblutung_vorhanden - 4,170 * White Matter Damage_vorhanden
(R= 0,547; F= 10,836; p< 0,00001).

**Patentansprüche**

1. Verfahren zur Detektion von saurem Gliafaserprotein (GFAP) im Blut eines früh- oder reifgeborenen Säugers, bei dem das Vorhandensein von GFAP mittels PCR-verstärktem Immunassay (I-PCR) detektiert wird und weiterhin kontinuierlich der Partialdruck von Stickstoffmonoxid im Atemgas des Säugers bestimmt wird.

2. Verfahren nach Anspruch 1, **dadurch gekennzeichnet, dass** das Vorhandensein von GFAP mittels Sandwich-I-PCR detektiert wird.

3. Verfahren nach Anspruch 1, **dadurch gekennzeichnet, dass** das Vorhandensein von GFAP mittels indirekter Sandwich-I-PCR detektiert wird.

4. Verfahren nach Anspruch 1, **dadurch gekennzeichnet, dass** das Vorhandensein von GFAP mittels indirekter I-PCR detektiert wird.

5. Verfahren nach Anspruch 1, **dadurch gekennzeichnet, dass** das Vorhandensein von GFAP mittels direkter I-PCR detektiert wird.

6. Verfahren nach einem der Ansprüche 1 bis 5, wobei weiterhin der Kopfumfang des Säugers bestimmt wird.

7. System zur Bestimmung von Hirnschäden bei Früh- und Reifgeborenen umfassend:

   a. ein Gerät zur Detektion von saurem Gliafaserprotein (GFAP) im Blut eines Säugers mittels PCR-verstärktem Immunassay (I-PCR) und
   b. ein Gerät zur Bestimmung des Partialdrucks von Stickstoffmonoxid im Atemgas eines Säugers.

8. System zur Bestimmung von Hirnschäden bei Früh- und Reifgeborenen nach Anspruch 7, weiterhin umfassend:
   c. ein Gerät zur Bestimmung des Kopfumfangs eines Säugers.

9. System zur Bestimmung von Hirnschäden bei Früh- und Reifgeborenen nach einem der Patentansprüche 7 bis 8, weiterhin umfassend eine Datenbank zur Erfassung und Verarbeitung der Daten.

**10.** System zur Bestimmung von Hirnschäden bei Früh- und Reifgeborenen nach Patentanspruch 9, wobei die Datenbank Mittel umfasst, um weitere vor, während und/oder nach der Geburt gewonnene Daten zu erfassen.

**11.** System zur Bestimmung von Hirnschäden bei Früh- und Reifgeborenen nach Patentanspruch 10, wobei die Datenbank Mittel umfasst, um weitere vor, während und/oder nach der Geburt gewonnene Daten zu erfassen, die eine Prognose zur weiteren psycho-motorischen Entwicklung bei Früh- und Reifgeborenen erlauben.

**Claims**

**1.** A method for the detection of acidic glial fiber protein (GFAP) in the blood of a premature or fullterm mammal, in which the presence of GFAP is detected by means of PCR-amplified immunoassay (I-PCR) and partial pressure of nitrogen monoxide is further continuously determined in the breath gas of the mammal.

**2.** Method as claimed in claim 1, **characterized in that** the presence of GFAP is detected by means of sandwich I-PCR.

**3.** Method as claimed in claim 1, **characterized in that** the presence of GFAP is detected by means of indirect sandwich I-PCR.

**4.** Method as claimed in claim 1, **characterized in that** the presence of GFAP is detected by means of indirect I-PCR.

**5.** Method as claimed in claim 1, **characterized in that** the presence of GFAP is detected by means of direct I-PCR.

**6.** Method as claimed in one of claims 1 to 5, wherein the head circumference of the mammal is further determined.

**7.** A system for determining brain damage in preterm and full-term infants, comprising:

a. an apparatus for detecting glial fiber protein (GFAP) in the blood of a mammal by means of PCR-amplified immunoassay (I-PCR) and
b. an apparatus for determining the partial pressure of nitrogen monoxide in the breath gas of a mammal.

**8.** System for determining brain damage in preterm and full-term infants as claimed in claim 7, further comprising:
c. an apparatus for determining the head circumference of a mammal.

**9.** System for determining brain damage in preterm and full-term infants as claimed in one of claims 7 to 8, further comprising a database for collecting and processing the data.

**10.** System for determining brain damage in preterm and full-term infants as claimed in claim 9, wherein the database comprises means to collect additional data obtained before, during and/or after birth.

**11.** System for determining brain damage in preterm and full-term infants as claimed in claim 10, wherein the database comprises means to collect additional data obtained before, during and/or after birth that allow to make a prognosis on the further psycho-motoric development in preterm and full-term infants.

**Revendications**

**1.** Procédé de détection de protéine acide fibrillaire gliale (GFAP) dans le sang d'un nourrisson prématuré ou né à terme, selon lequel on détecte la présence de GFAP au moyen d'un immunoessai renforcé par PCR (I-PCR) et on détermine en outre de façon continue la pression partielle de monoxyde d'azote dans le gaz respiratoire du nourrisson.

**2.** Procédé selon la revendication 1, **caractérisé en ce que** la présence de GFAP est détectée à l'aide d'une I-PCR emboîtée.

**3.** Procédé selon la revendication 1, **caractérisé en ce que** la présence de GFAP est détectée à l'aide d'une I-PCR emboîtée indirecte.

**4.** Procédé selon la revendication 1, **caractérisé en ce que** la présence de GFAP est détectée à l'aide d'une I-PCR indirecte.

**5.** Procédé selon la revendication 1, **caractérisé en ce que** la présence de GFAP est détectée à l'aide d'une I-PCR directe.

**6.** Procédé selon l'une des revendications 1 à 5, selon lequel on détermine en outre le périmètre crânien du nourrisson.

**7.** Système pour déterminer des lésions cérébrales chez les enfants prématurés et nés à terme, comprenant

> a. un appareil pour détecter la protéine acide fibrillaire gliale (GFAP) dans le sang d'un nourrisson, à l'aide d'un immunoessai renforcé par PCR (I-PCR), et
> b. un appareil pour déterminer la pression partielle de monoxyde d'azote dans le gaz respiratoire d'un nourrisson.

**8.** Système pour déterminer des lésions cérébrales chez les enfants prématurés et nés à terme, selon la revendication 7, comprenant en outre :
c. un appareil pour déterminer le périmètre crânien d'un nourrisson.

**9.** Système pour déterminer des lésions cérébrales chez les enfants prématurés et nés à terme, selon l'une des revendications 7 à 8, comprenant en outre une base de données pour recueillir et traiter les données.

**10.** Système pour déterminer des lésions cérébrales chez les enfants prématurés et nés à terme, selon la revendication 9, dans lequel la base de données comprend des moyens pour recueillir des données supplémentaires, obtenues avant, pendant et/ou après la naissance.

**11.** Système pour déterminer des lésions cérébrales chez les enfants prématurés et nés à terme, selon la revendication 10, dans lequel la base de données comprend des moyens pour recueillir des données supplémentaires, obtenues avant, pendant et/ou après la naissance, qui permettent d'établir un pronostic concernant le développement psychomoteur d'enfants prématurés et nés à terme.

Bestimmung des Kopfumfangs

Bestimmung von
NO im Atemgas

Bestimmung von saurem Gliafaserprotein

**Datenbank**
Risiken, Befunde,
Bildgebung vor, während
und nach der Geburt, Behandlung mit Stammzellen
aus Nabelschnurblut, Nachsorgeuntersuchungen

FIG. 1

FIG. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10130296 B4

**In der Beschreibung aufgeführte Nicht-Patentliteratur**

- **L. PAPA et al.** Performance of Glial Fibrillary Acidic Protein in Detecting Traumatic Intracranial Lesions on Computed Tomography in Children and Youth With Mild Head Trauma. *Academic emergency medicine: official journal of the Society for Academic Emergency Medicine,* November 2015, vol. 22 (11), 1274-1282
- **T. BOGOSLOVSKY et al.** Fluid Biomarkers of Traumatic Brain Injury and Intended Context of Use. *Diagnostics,* 2016, vol. 6, 37
- **BERGER et al.** *Eur. J. Obstet. Gynaecol. Reprod. Biol.,* 1997, vol. 75, 191-203
- **R. BERGER et al.** *Creatine protects the immature brain from hypoxic-ischemic injury*
- **MIDDELANIS et al.** *J. Soc. Gynecol. Investig.,* 2003, vol. 10 (2
- **A. JENSEN.** Stammzellen aus Nabelschnurblut heilen kindlichen Hirnschaden. *Top Magazin Ruhr, Wissenschaft - Medizin,* 2009, vol. 23 (4), 80-81
- **A. JENSEN.** Erste Therapie eines kindlichen hypoxischen Hirnschadens mit Zerebralparese nach Herzstillstand? - Heilversuch durch autologe Nabelschnurstammzell-Transplantation. *Regenerative Medizin,* 2011, vol. 4 (1), 30-31
- **A. JENSEN et al.** Perinatal brain damage - from neuroprotection to neuro regeneration using cord blood stem cells. *Med. Klein.,* 2003, vol. 98 (2), 22-26
- **C. MEIER et al.** Spastic paresis after perinatal brain damage in rats is reduced by human cord blood mononuclear cells. *Pediatr. Res.,* 2006, vol. 59 (2), 244-249
- **LV et al.** Neonatal hypoxic ischemic encephalopathy-related biomarkers in serum and cerebrospinal fluid. *CLINICA CHIMICA ACTA,* 2015, vol. 450, 282-297
- **STEWART et al.** 445: Serum glial fibrillary acidic protein levels are significantly elevated in preterm neonates with neurologic morbidity and mortality. *AMERICAN JOURNAL OF OBSTETRICS & GYNECOLOGY,* Januar 2011, vol. 204 (1), S179
- **KUCZIUS et al.** Simultaneous detection of three CNS indicator proteins in complex suspensions using a single immuno-PCR protocol. *ANAL BIOCHEM,* 2012, vol. 431 (1), 4-10
- **KUCZIUS et al.** High sensitivity detection of the glial fibrillary acidic protein as indicator for TSE risk material in meat products using an immuno-PCR. *MOLECULAR NUTRITION & FOOD . RESEARCH,* 2009, vol. 53 (10), 1329-1335
- **NIEMEYER et al.** Immuno-PCR: high sensitivity detection of proteins by nucleic acid amplification. *TRENDS IN BIOTECHNOLOGY,* 2005, vol. 23 (4), 208-216
- **MARKS.** Hypoxic-ischemic brain injury and neuroprotection in the newborn infant. *FUTURE NEUROL,* 2013, vol. 8 (3), 303-319
- **GROW et al.** Pathogenesis of hypoxic-ischemic cerebral injury in the term infant: current concepts. *CLINICS IN PERINATOLOGY,* 2002, vol. 29 (4), 585-602
- **KAUR et al.** Hypoxie damage to the periventricular white matter in neonatal brain: role of vascular endothelial growth factor, nitric oxide and excitotoxicity. *J NEUROCHEM,* 2006, vol. 98 (4), 1200-1216
- **P.K. METHA et al.** Detection of potential microbial antigens by immuno-PCR (PCR-amplified immunoassay). *Journal of Medical Microbiology,* 2014, vol. 63, 627-641